# EUROPÄISCHE PATENTSCHRIFT

(11) **EP 3 897 468 B1**
(45) Veröffentlichungstag und Bekanntmachung des Hinweises auf die Patenterteilung: **21.05.2025**
(21) Anmeldenummer: 19820731.8
(22) Anmeldetag: 10.12.2019
(51) Int. Cl.: A61F 2/64, A61F 2/68, A61F 5/01, A61F 2/50

(54) **PROTHESENEINRICHTUNG**
PROSTHETIC DEVICE
DISPOSITIF PROTHÉTIQUE

(30) Priorität: 20.12.2018 DE 102018133103
(43) Veröffentlichungstag der Anmeldung: 27.10.2021
(73) Patentinhaber: Ottobock SE & Co. KGaA, 37115 Duderstadt (DE)
(72) Erfinder: PUSCH, Martin, 37115 Duderstadt (DE)
(74) Vertreter: Gramm, Lins & Partner Patent- und Rechtsanwälte PartGmbB
(86) Internationale Anmeldenummer: PCT/EP2019/084495
(87) Internationale Veröffentlichungsnummer: WO 2020/126695

(56) Entgegenhaltungen:
- JP-A- 2009 232 999
- US-B1- 6 808 540

## Beschreibung

Die Erfindung betrifft eine Protheseneinrichtung für eine untere Extremität mit einem Prothesenkniegelenk mit einem Oberteil, an dem eine proximale Prothesenkomponente angeordnet ist, mit einem Unterteil, das mit dem Oberteil um eine Kniegelenksachse schwenkbar verbunden ist, und mit einer distalen Prothesenkomponente, an der ein Prothesenfuß festlegbar ist. Die proximale Prothesenkomponente kann insbesondere als Prothesenschaft ausgebildet sein, der über einen ersten Anschluss, der an dem Oberteil angeordnet oder ausgebildet ist, an dem Prothesenkniegelenk befestigt oder befestigbar ist.

Prothesenkniegelenke ersetzen natürliche Kniegelenke und ermöglichen eine Verschwenkung eines Unterschenkelteils, das überwiegend aus einem Unterschenkelrohr und einem daran befestigten Prothesenfuß besteht, relativ zu dem Oberschenkel oder einem Oberschenkelteil. In der Regel werden Prothesenkniegelenke an einem Oberschenkelstumpf festgelegt. Dazu wird ein sogenannter Prothesenschaft an einem proximalen ersten Anschluss an dem Prothesenkniegelenk verbunden. Der Prothesenschaft nimmt den Oberschenkelstumpf auf und legt die weitere Prothese an dem Patienten fest. Dazu kann der Prothesenschaft als starrer Schaft ausgebildet sein, der über Gurte oder über ein Unterdrucksystem oder ein sogenanntes Linersystem an dem Stumpf oder Patienten gehalten wird. Grundsätzlich sind auch andere Prothesenschaftausgestaltungen möglich.

Die einfachste Form eines Prothesenkniegelenkes ist ein einachsiges Prothesenkniegelenk, das eine freie Verschwenkung um eine einfache Achse ermöglicht. Darüber hinaus existieren polyzentrische Prothesenkniegelenke, mit denen komplexe, relative Schwenkbewegungen von Oberteil zu Unterteil erreicht werden können. Den Prothesenkniegelenken können Dämpfungseinrichtungen, einstellbare Endanschläge, Antriebe, Bremsen oder Verriegelungseinrichtungen zugeordnet sein, um eine Anpassung an die Erfordernisse während der Benutzung zu erfüllen.

Bei motorisch nicht angetriebenen Prothesenkniegelenken findet eine Beeinflussung der Standphasenflexion über Dämpfungseinrichtungen oder Federeinrichtungen statt.

Aus der EP 0 439 028 A1 ist ein polyzentrisches Prothesenkniegelenk bekannt, bei dem eine Polyzentrik um eine Schwenkachse entgegen einer Feder- oder Dämpferkraft verschwenkt werden kann, so dass es möglich ist, bei Fersenlast zu Beginn der Standphase des Gehens ein Nachgeben zu ermöglichen. Dazu wird die gesamte Polyzentrik verlagert, ohne dass die Polyzentrik in sich verändert wird.

Die JP 2009-232999 A betrifft ein Prothesenkniegelenk mit einem Oberteil und einem Unterteil, die gelenkig um eine Schwenkachse aneinander gelagert sind. Eine Widerstandseinrichtung ist an einem Exzenter abgestützt, der zusammen mit dem Oberteil um die Schwenkachse verschwenkt wird. Bei zunehmendem Verschwenkwinkel wird die Widerstandseinrichtung zunehmend linear verlagert. Über die Kontur des Exzenters kann die Verlagerungsgeschwindigkeit der Widerstandseinrichtung verändert werden.

Die US 6 808 540 B1 betrifft ein polyzentrisches Kniegelenk mit einem Oberteil und einem schwenkbar daran gelagerten Unterteil. Innerhalb des Unterteils ist ein Hydraulikdämpfer zusammen mit einem Federelement angeordnet. Über eine Rolle, die mit dem Federelement über den Hydraulikzylinder gekoppelt ist, wird eine Druckkraft auf einen Steuernocken ausgeübt. Wird das Prothesenkniegelenk eingebeugt, verlagert sich der Steuernocken und ein Widerstand wird über den Hydraulikdämpfer in Verbindung mit dem elastischen Element bereitgestellt. Bei zunehmender Verschwenkung des Oberteils wird ein zunehmend größerer Widerstand gegen eine Flexion bereitgestellt.

Die Flexion entgegen einem Dämpfungs- und Federwiderstand in der Standphase ist vergleichsweise gering und unabhängig von der Gehgeschwindigkeit. Bei einem Gehen in der Ebene erfolgt bei einem menschlichen Kniegelenk nach dem Fersenkontakt bei zunehmender Gehgeschwindigkeit eine zunehmende Kniebeugung. Herkömmliche Prothesenkniegelenke können diesen Effekt nicht abbilden, da das Hüftstreckmoment bei einer zunehmenden Gehgeschwindigkeit stark ansteigt und dem Einbeugeeffekt entgegenwirkt.

Aufgabe der vorliegenden Erfindung ist es daher, eine Protheseneinrichtung bereitzustellen, mit der ein Gehen mit weniger Aufwand möglich ist.

Erfindungsgemäß wird diese Aufgabe mit einer Protheseneinrichtung mit den Merkmalen des Hauptanspruches gelöst. Vorteilhafte Ausgestaltungen der Erfindung sind in den Unteransprüchen, der Beschreibung sowie den Figuren offenbart.

Die erfindungsgemäße Protheseneinrichtung für eine untere Extremität mit einem Prothesenkniegelenk mit einem Oberteil, an dem eine proximalen Prothesenkomponente angeordnet ist, einem Unterteil, das mit dem Oberteil um eine Kniegelenksachse schwenkbar verbunden ist, und einer distalen Prothesenkomponente, an der ein Prothesenfuß festlegbar ist, sieht vor, dass die distale Prothesenkomponente durch eine in Längserstreckung der distalen Prothesenkomponente wirkende Axialkraft in Richtung auf die Kniegelenksachse verschieblich gelagert ist und eine Kraftübertragungseinrichtung dem Kniegelenk zugeordnet ist, die in der Standphase eine Verschiebung der distalen Prothesenkomponente in Richtung auf die Kniegelenksachse und somit eine Längenveränderung der distalen Prothesenkomponente in ein Flexionsmoment um die Kniegelenksachse umwandelt. Durch die Längsverschieblichkeit der distalen Prothesenkomponente ist es möglich, eine Axialkraft, die in Längsrichtung dieser distalen Prothesenkomponente wirkt, umzuwandeln in ein Flexionsmoment, das um die Kniegelenksachse wirkt. Dazu ist eine Kraftübertragungseinrichtung sowohl dem Kniegelenk als auch der distalen Prothesenkomponente zugeordnet und mit beiden Komponenten gekoppelt, so dass die stauchende Kraft, beispielsweise beim dem sogenannten Fersenstoß oder Heel Strike, in eine Flexionsunterstützung umgewandelt wird. Die Umwandlung kann direkt erfolgen, beispielsweise über eine mechanische Kraftübertragungseinrichtung, die als Seilzug, Pleuelstange oder Kurvenbahn ausgebildet sein kann oder über eine hydraulische Kraftübertragungseinrichtung, die Vorteile hinsichtlich der Kraftübertragungen hat. Beide Kraftübertragungseinrichtungen, sowohl eine mechanische als auch eine hydraulische, können mit einer Getriebeeinrichtung, einer Umlenkeinrichtung oder einem Übersetzungselement versehen sein, um eine Kraftübersetzung oder eine Wegübersetzung zu ermöglichen. Dadurch können Kraftrichtungen umgelenkt, Wegverlängerungen oder Wegverkürzungen für die Aufbringung eines Flexionsmomentes und Kraftveränderungen oder Begrenzungen erreicht werden, was ohne eine zwischen der distalen Komponente und dem Kniegelenk angeordnete Einrichtung nicht möglich wäre.

Die distale Prothesenkomponente ist bevorzugt teleskopierbar ausgebildet oder verschieblich an dem Unterteil gelagert, um bei einem Auftreten oder einer Belastung in Axialrichtung der distalen Komponente eine Längenveränderung bereitstellen zu können, die dann in ein Flexionsmoment um die Kniegelenksachse umgesetzt wird.

Die distale Prothesenkomponente kann über ein Federelement gegen eine Verschiebung vorgespannt gelagert sein, um während der Schwungphase oder während einer statischen Belastung, beispielsweise beim Stehen, keine Längenveränderung oder nur eine geringe Längenveränderung auszuführen, so dass über die Dimensionierung des Federelementes erreicht werden kann, dass nur bei bestimmten Belastungssituationen ein Flexionsmoment um die Kniegelenksachse ausgeübt wird. Die Federvorspannung kann einstellbar ausgebildet sein, insbesondere motorisch einstellbar, um auch während der Benutzung der Protheseneinrichtung eine Anpassung an die jeweiligen Benutzungssituationen oder an die jeweiligen Wünsche des Prothesennutzers vornehmen zu können. Die Federvorspannung kann auch so hoch gesetzt werden, dass eine effektive Längenveränderung nicht möglich ist. Ebenso ist es möglich, dass eine Sperreinrichtung der distalen Prothesenkomponente zugeordnet ist, um die Längsverschieblichkeit und damit die Längenveränderbarkeit und das Aufbringen eines Flexionsmomentes zu unterbinden.

Das Federelement kann einen progressiven Widerstand einer Verschiebung der distalen Prothesenkomponente in Axialrichtung entgegensetzen, wodurch das maximale Flexionsmoment begrenzt werden kann.

Dem Kniegelenk kann eine Energiespeichereinrichtung zur Speicherung von Flexionsenergie zugeordnet sein. Die Energiespeichereinrichtung kann Energie während des Flexionsvorganges speichern und der Gelenkeinrichtung wieder zuführen, um eine Extensionsbewegung zu unterstützen. Auf diese Weise kann eine Flexionsunterstützung durch die Kraftübertragungseinrichtung entgegen einer Feder oder einer Energiespeichereinrichtung erfolgen, wobei die Energiespeichereinrichtung einen progressiven Widerstand einer Flexion entgegensetzen kann. In der Energiespeichereinrichtung wird diese Flexionsenergie gespeichert und kann der Gelenkeinrichtung wieder zugeführt werden, um eine Extensionsbewegung zu unterstützen. Dies ist insbesondere bei der Standphasenextension sinnvoll, um nach dem Einbeugen des Prothesenkniegelenkes unmittelbar nach dem Heel Strike die Extensionsbewegung zu erleichtern und diese nicht ausschließlich über die Hüftmuskulatur ausführen lassen zu müssen.

In einer Weiterbildung der Erfindung ist es vorgesehen, dass die Energiespeichereinrichtung als ein Federelement, insbesondere als ein Federelement mit einer sich über den Kniewinkel ändernden Federsteifigkeit ausgebildet ist. Das Federelement kann beispielsweise als eine Knickfeder ausgebildet sein, die in Extensionsrichtung wirkt. Insbesondere bei der Standphasenbeugung nimmt der Abstand zwischen dem Kraftvektor der Bodenreaktionskraft und der Kniegelenksachse zu. Das Federelement oder die Extensionsfeder zeigt eine zunehmende Nachgiebigkeit mit einem zunehmenden Beugewinkel, also mit einem sich verringernden Kniewinkel. Dadurch wird erreicht, dass der Aufsetzimpuls beim Heel Strike bewirkt, dass das Federelement komprimiert oder verformt wird und bei einer zunehmenden Einbeugung des Kniegelenkes eine sich verringernde Rückstellkraft bereitgestellt wird, um ein sich verringerndes oder zumindest nicht erhöhendes Flexionsmoment aufgrund des sich vergrößernden Abstandes zwischen dem Kraftvektor der Bodenreaktionskraft und der Kniegelenkachse bereitzustellen. Bei einem großen Beugewinkel wird mit der degressiven Federcharakteristik des Federelementes eine größere Nachgiebigkeit hinsichtlich der Kniebeugung erreicht. Wird während des Gehens in der Standphase der resultierende Vektor der Bodenreaktionskraft wieder in Richtung auf die Knieachse und in Gehrichtung vor die Knieachse verlagert, extendiert das Kniegelenk und die Extension wird durch die sich entspannende Feder unterstützt. Je gestreckter das Knie dabei ist, desto größer ist die Unterstützungswirkung des Federelementes. Durch eine geeignete Abstimmung des Federelementes ist es möglich, eine Stoßdämpfung beim Gehen durch die Kniebeugung zu realisieren, statt durch eine axiale Dämpfereinrichtung, ohne dass eine gewollte Energiedissipation durch Umwandlung kinetischer Energie in Wärme erfolgt. Die Federkennlinie des Federelementes kann zunächst ansteigen und ab einem bestimmten Deformationsgrad wieder abfallen.

Der Energiespeichereinrichtung kann eine Aktivierungs- und/oder Deaktivierungseinrichtung zugeordnet sein. Dadurch ist es möglich, für unterschiedliche Bewegungssituationen ein Rückstellelement und eine Extensionsunterstützung bereitzustellen und für andere Bewegungssituationen eine solche auszuschließen.

In einer Weiterbildung der Erfindung ist es vorgesehen, dass dem Kniegelenk zumindest ein Dämpfer zur Dämpfung der Verschwenkbewegung um die Kniegelenksachse zugeordnet ist. Alternativ oder ergänzend ist es vorgesehen, dass zumindest ein Dämpfer zur Dämpfung der Verschiebebewegung der distalen Prothesenkomponente in Richtung auf die Gelenksachse zugeordnet ist. Über die Dämpfung mit dem Dämpfer ist es möglich, eine herkömmliche Standphasen- und/oder Schwungphasendämpfung zusätzlich zu einer Flexionsunterstützung beim Auftreten einer Axialkraft bereitzustellen. Dadurch ist es möglich, die Protheseneinrichtung auch während der Schwungphase hinsichtlich ihres Extensions- oder Flexionsverhaltens zu beeinflussen. Ein Dämpfer zur Beeinflussung der Verschiebebewegung ermöglicht eine Anpassung des Verhaltens der Flexionsunterstützung und der Art und Weise sowie des Umfanges der Aufbringung des Flexionsmomentes.

Der zumindest eine Dämpfer ist bevorzugt einstellbar ausgebildet, um eine Anpassung an das jeweilige Nutzerverhalten oder an die Bedürfnisse des jeweiligen Patienten vornehmen zu können. Die Dämpfereinrichtung zur Dämpfung der Verschwenkbewegung um die Kniegelenksachse kann mit der Dämpfereinrichtung zur Dämpfung der Verschiebebewegung gekoppelt sein. Ebenso ist es möglich, dass der Dämpfer zur Dämpfung der Verschwenkbewegung dergestalt mit einem Fluidreservoir in der distalen Prothesenkomponente gekoppelt ist, dass bei einer Verschiebung der distalen Prothesenkomponente in Richtung auf die Kniegelenkachse ein Hydraulikfluid in eine Extensionskammer oder Flexionskammer geleitet wird, je nachdem, welche Bewegung unterstützt oder welcher Bewegung entgegen gewirkt werden soll. Der Zuleitung kann ein Schaltventil zugeordnet sein, das das durch die Verschiebebewegung komprimierte Hydraulikfluid entweder in die Extensionskammer oder die Flexionskammer leitet, je nach Gangsituation oder gewünschtem Verhalten der Protheseneinrichtung.

Dem Kniegelenk kann ein schaltbarer Anschlag oder eine Bremse zugeordnet sein, der oder die eine Flexion um die Kniegelenksachse sperrt oder bremst. Der Anschlag kann in Abhängigkeit von der jeweiligen Bewegungsphase aktiviert oder deaktiviert werden. Wird über eine Sensoreinrichtung erkannt, dass sich die Protheseneinrichtung in der Standphase befindet, beispielsweise über einen Kraftsensor in einem Prothesenfuß, wird ein Anschlag aktiviert, der eine Maximalflexion begrenzt. Entsprechende Maßnahmen können über eine Bremse erfolgen, über die eine Flexionsbewegung ab Erreichen eines bestimmten Kniewinkels abgebremst wird. Befindet sich der Patient mit dem versorgten Bein in der Schwungphase, was beispielsweise ebenfalls über eine Kraftsensor in der Fußsohle oder einen anderen Axialkraftsensor ermittelt wird, wird der Flexionsanschlag oder die Flexionsbremse deaktiviert oder gelöst, so dass eine Schwungphasenflexion ungehindert oder ungebremst stattfinden kann. Erst zu einem wesentlichen späteren Zeitpunkt bei Erreichen eines maximalen Flexionswinkels von ca. 60° kann ein solcher Flexionsanschlag oder eine Bremse aktiviert werden, um eine zu große Flexion während der Schwungphase zu verhindern.

Eine Weiterbildung der Erfindung sieht vor, dass zumindest ein Sensor, insbesondere zur Erfassung der Axialkraft, des Axialkraftverlaufes, der Winkelstellung, der Raumlage oder der Beschleunigungen zumindest einer Komponente der Protheseneinrichtung, mit einer Steuerungseinrichtung gekoppelt ist, die einen Antrieb des Anschlages oder der Bremse aktiviert oder deaktiviert.

Nachfolgend werden Ausführungsbeispiele der Erfindung anhand der beigefügten Figuren näher erläutert. Es zeigen:
- Figur 1: - eine schematische Darstellung einer Protheseneinrichtung;
- Figur 2: - eine schematische Darstellung einer Protheseneinrichtung mit Kraftübertragungseinrichtung;
- Figuren 3 - 5: - Varianten der Figur 2; sowie
- Figur 6: - unterschiedliche Kniewinkelverläufe;
- Figur 7: - eine Variante der Figur 2 mit Energiespeicher;
- Figur 8: - eine Variante der Figur 2 mit Sensoren;
- Figur 9 stütz-: - Kniewinkelverläufe bei unterschiedlichen Extensionsunterungen; sowie
- Figur 10: - Darstellungen von Federkennlinien.

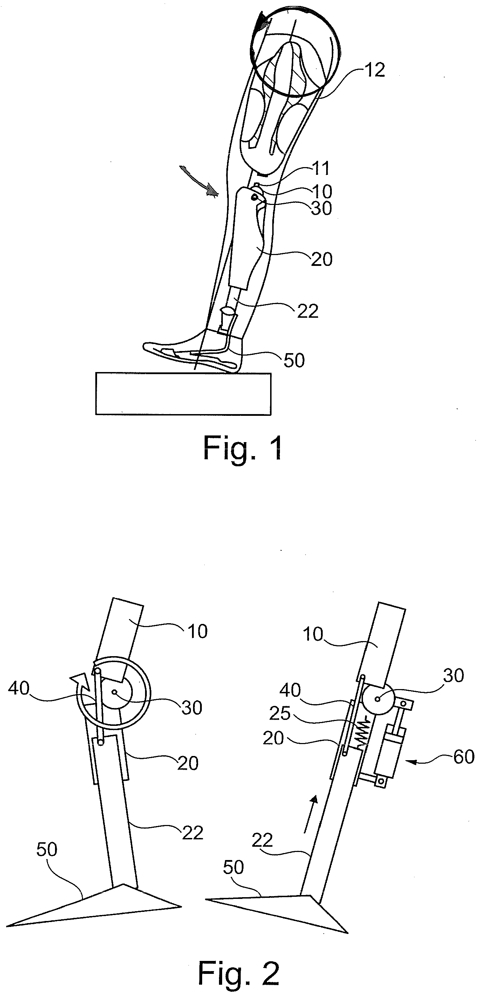

In der Figur 1 ist in einer schematischen Darstellung eine Protheseneinrichtung für eine untere Extremität in Gestalt eines Prothesenbeines gezeigt, das ein Prothesenkniegelenk aufweist, das ein Oberteil 10 aufweist, an dem ein erster, proximaler Anschluss 11 in Gestalt eines Pyramidenadapters angeordnet ist. An dem proximalen ersten Anschluss 11 kann eine proximale Prothesenkomponente 12 in Gestalt eines Prothesenschaftes zur Aufnahme eines Oberschenkelstumpfes angeordnet werden. Der Oberschenkelschaft kann über eine Saugschaftechnologie oder über andere Festlegungsmechanismen an dem Stumpf des Patienten festlegbar sein. An dem Oberteil 10 ist eine Kniegelenksachse 30 ausgebildet, um die herum ein Unterteil 20 des Prothesenkniegelenkes schwenkbar befestigt ist. Das Unterteil 20 ist im dargestellten Ausführungsbeispiel als ein Aufnahmerahmen ausgebildet, in dem eine hydraulische Dämpfereinrichtung angeordnet ist, die während des Gehens hinsichtlich der Dämpfungseigenschaften verstellbar ausgebildet ist. Distal zu dem Unterteil 20 ist eine distale Prothesenkomponente 22 in Gestalt eines Unterschenkelrohres angeordnet, wobei das Unterschenkelrohr ebenfalls Aufnahmeeinrichtungen zum Befestigen eines Prothesenfußes 50 an der distalen Prothesenkomponente 22 aufweist.

Protheseneinrichtungen für eine untere Extremität mit einem Prothesenkniegelenk weisen grundsätzlich diesen Aufbau auf, wenn ein modularer Aufbau vorhanden ist, also der Prothesenschaft 12 separat von dem Prothesenkniegelenk eines solchen hergestellt und über einen Anschluss 11 daran befestigbar ist. Es besteht auch die Möglichkeit, dass die proximale Prothesenkomponente 12 zur Aufnahme eines Prothesenstumpfes unmittelbar mit dem Oberteil 10 ausgebildet ist, so dass neben der Ausbildung einer Schwenkachse 30 zwischen Oberteil 10 und Unterteil 20 gleichzeitig die Aufnahmefunktion für den Stumpf verwirklicht wird. Zudem besteht auch die Möglichkeit, dass statt eines separaten Prothesenfußes 50 dieser unmittelbar an der distalen Prothesenkomponente 22 oder dem Unterschenkelrohr ausgebildet ist. Ein modularer Aufbau hat jedoch den Vorteil, dass die Protheseneinrichtung kostengünstiger hergestellt werden kann und eine Verwendung eines Komponententyps für mehrere unterschiedliche Anwender erfolgen kann.

Beim Gehen in der Ebene ist es zur Erhöhung der Gehgeschwindigkeit in der Regel notwendig, ein erhöhtes Hüftstreckmoment aufzubringen, was durch den oberen Kreis dargestellt ist. Dieses Hüftstreckmoment wird in der Standphase aufgebracht, um den Körperschwerpunkt möglichst schnell vor das jeweilige Bein zu bewegen. Dieses Hüftstreckmoment in der Standphase wirkt sich jedoch gleichzeitig stabilisierend auf das Kniegelenk aus, das heißt, dass ein Extensionsmoment um die Kniegelenksachse 30 wirkt. Die Kraftwirkung ist durch den frontalen Pfeil angedeutet. Bei einem menschlichen Kniegelenk wird bei einer erhöhten Gehgeschwindigkeit und einem erhöhten Hüftstreckmoment über eine entsprechende Muskelbewegung eine erhöhte Kniebeugung oder Flexion mehr oder weniger unwillkürlich während der Standphase ausgeführt. Eine erhöhte Gehgeschwindigkeit und ein damit einhergehendes erhöhtes Hüftstreckmoment führen somit zu einem erhöhten Standphasenflexionswinkel und einem insgesamt "weicheren" Gang. Bei Protheseneinrichtungen wirkt sich jedoch ein erhöhtes Hüftstreckmoment stabilisierend und extendierend auf das Kniegelenk aus, so dass Prothesenkniegelenke weniger flektieren, wenn die Gehgeschwindigkeit erhöht wird. Dies führt zu einer verringerten Standphasenflexion und zu einem unphysiologischen, "härteren" Gang bei zunehmender Gehgeschwindigkeit.

Eine erfindungsgemäße Protheseneinrichtung ist schematisch in der Figur 2 in zwei Zuständen dargestellt. In dem rechten Zustand befindet sich die Protheseneinrichtung in einem maximal extendierten Zustand unmittelbar vor dem Aufsetzen auf den Boden, in der linken Darstellung in einer Stellung am Ende der Standphase. Sowohl das Oberteil 10 als auch das Unterteil 20 sind rein schematisch dargestellt, ebenso die Kniegelenksachse 30, um die das Unterteil 20 relativ zu dem Oberteil 10 verschwenkt gelagert ist. Neben einem monozentrischen Kniegelenk kann auch ein polyzentrisches Kniegelenk zwischen dem Oberteil 10 und dem Unterteil 20 angeordnet oder ausgebildet sein. Das Oberteil 10 gemäß dieser Ausführungsform kann einen Adapter oder Anschlussmittel 11 aufweisen, um mit einem separat gefertigten Prothesenschaft oder einer anderen proximalen Prothesenkomponente 12 verbunden zu werden.

Das Unterteil 20 ist ebenfalls nur schematisch dargestellt, andere Formen des Prothesenkniegelenkunterteils 20 können ausgebildet sein.

Distal zu dem Unterteil 20 ist eine distale Prothesenkomponente 22 in Gestalt eines Unterschenkelrohrs in Längsrichtung der distalen Prothesenkomponente 22 verschieblich angeordnet. Die Längsverschieblichkeit der distalen Prothesenkomponente 22 relativ zu dem Unterteil 20 ist durch den Pfeil in Richtung auf die Kniegelenksachse 30 angedeutet. An der distalen Prothesenkomponente 22 ist der schematisch dargestellte Prothesenfuß 50 befestigt.

Zwischen der distalen Prothesenkomponente 22 und dem Oberteil 10 ist eine Kraftübertragungseinrichtung 40 in Gestalt einer Pleuelstange angeordnet, die zugkraftübertragend und druckkraftübertragend gelenkig sowohl an dem Oberteil 10 als auch an der distalen Prothesenkomponente 22 angeordnet ist. Wird nun die distale Prothesenkomponente 22 relativ zu dem Unterteil 20 in Richtung auf die Kniegelenksachse 30 verlagert, wird eine Druckkraft über die Kraftübertragungseinrichtung 40 von der distalen Prothesenkomponente 22 auf das Oberteil 10 übertragen. Der Anlenkungspunkt der Kraftübertragungseinrichtung 40 an dem Oberteil 10 befindet sich in Gehrichtung vor der Kniegelenksachse 30, so dass die Druckkraft, die von dem Prothesenfuß 50 über die distale Prothesenkomponente 22 und die Kraftübertragungseinrichtung 40 auf das Oberteil 10 ausgeübt wird, in ein Flexionsmoment umgewandelt wird, das zu einer Einbeugung oder zumindest zu einer Unterstützung einer Einbeugung des Prothesenkniegelenkes um die Kniegelenksachse 30 führt. Die linke Darstellung der Figur 2 zeigt, dass die distale Prothesenkomponente 20 bereits in die hülsenartige Aufnahme in dem Unterteil 20 eingeschoben worden ist, so dass sich über die Kraftübertragungseinrichtung 40 in Gestalt einer Pleuelstange eine Verschwenkbewegung des Oberteils 10 relativ zu dem Unterteil 20 ergeben hat. Der gebogene Pfeil deutet das Flexionsmoment und die Wirkrichtung der aufgebrachten Druckkraft von der Kraftübertragungseinrichtung 40 auf das Oberteil 10 an.

In der rechten Darstellung der Figur 2 ist zudem ein Energiespeicher in Gestalt einer Feder 25 zwischen der distalen Prothesenkomponente 22 und dem Unterteil 20 angeordnet. Die Feder 25 kann, wie dargestellt, als eine Spiralfeder oder Wendelfeder oder ein anderes, Druckkräfte aufnehmendes Element ausgebildet sein und bei einer Axialbelastung komprimiert werden. Wird die distale Prothesenkomponente 22 beim Fersenstoß oder während der Standphase in Richtung auf die Knieachse 30 relativ zu dem Unterteil 20 verlagert, wird das Federelement 25 vorgespannt. Bei einer sich reduzierenden Belastung in Axialrichtung, beispielsweise am Ende der Standphase oder in der Schwungphase, entspannt sich das Federelement 25 und bewegt die distale Komponente 22 wieder in die Ausgangsstellung von der Knieachse 30 weg. Dadurch wird auch die Extensionsbewegung des Unterteils 20 relativ zu dem Oberteil 10 unterstützt, da die Drucckraft des komprimierten Federelementes 25 über die Kopplungseinrichtung 40 in eine Extensionsbewegung des Unterteils 20 umgewandelt wird.

An der Rückseite der Protheseneinrichtung ist schematisch ein Dämpfer 60 dargestellt. Der Dämpfer 60 kann auch innerhalb des Unterteils 20 in Kombination mit dem Federelement 25 angeordnet sein. Über den Dämpfer 60 ist es möglich, die Axialverlagerung der distalen Prothesenkomponente 22 zu beeinflussen. Der Dämpfer 60 ist vorteilhafterweise einstellbar, um so das Verlagerungsverhalten bei einer Kompression sowie bei einer Entspannung des Federelements 25 zu beeinflussen. Über diese Veränderung der Verhaltensweisen der Entspannung und/oder Kompression werden gleichzeitig auch die Flexionsunterstützung und die Extensionsbewegung bei Entlastung beeinflusst. Das Federelement 25 kann auch extern außerhalb des Unterteils 20 in der Dämpfereinrichtung 60 angeordnet sein.

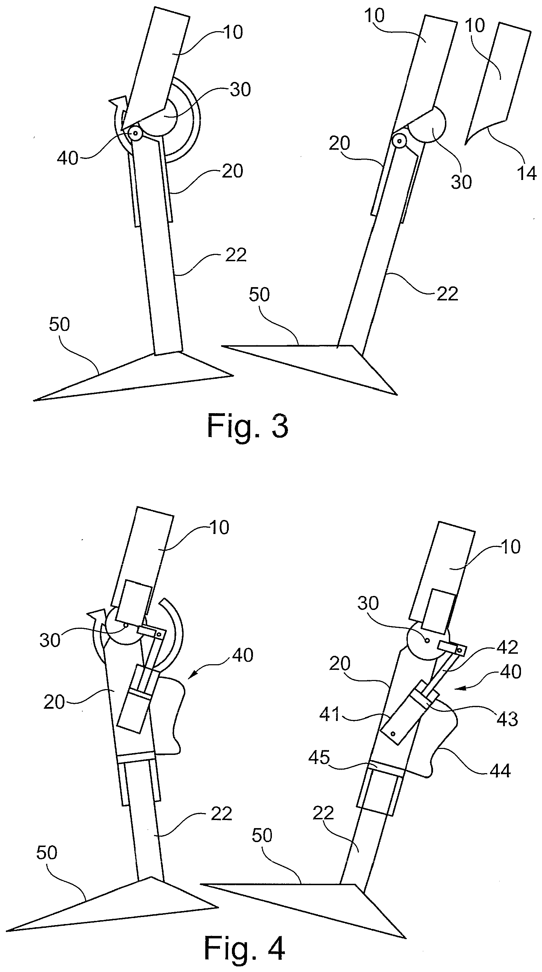

Eine Variante der Kraftübertragungseinrichtung 40 ist in der Figur 3 dargestellt. Der grundsätzliche Aufbau der Protheseneinrichtung entspricht dem der Figur 2. Statt einer Pleuelstange ist die Kraftübertragungseinrichtung 40 in dem Ausführungsbeispiel der Figur 3 als eine Kurvenbahn ausgestaltet. Die Kraftübertragungseinrichtung 40 ist in dem dargestellten Ausführungsbeispiel als eine Rolle ausgebildet, die an dem proximalen Ende der distalen Prothesenkomponente 22 angeordnet ist. Über die Rolle 40 oder über einen Gleitkörper wird ein Kontakt zu einer Lauffläche 14 oder einer Gleitfläche 14 auf der distalen Stirnseite des Oberteils 10 oder auf einer entsprechend angeordneten Lauffläche 14 oder Gleitfläche 14 ausgeübt. In der rechten Darstellung der Figur 3 sind zwei unterschiedliche Konturen von Laufflächen oder Gleitflächen 14 gezeigt, die linke Kontur ist geradlinig, die rechte Kontur weist einen kurvenförmigen Verlauf auf. Wird die distale Prothesenkomponente 22 in Richtung auf die Kniegelenksachse 30 relativ zu dem Unterteil 20 verschoben, gleitet oder rollt die Kraftübertragungseinrichtung 40 auf der Gleit- oder Lauffläche 14 ab. Der Kontaktpunkt zwischen der Kraftübertragungseinrichtung 40 und der Lauffläche 14 oder Gleitfläche 14 liegt dabei vor der Kniegelenksachse 30, so dass bei einem Auftreten und einer Relativverlagerung der distalen Prothesenkomponente 22 in Richtung auf die Kniegelenksachse 30 eine Kraft auf das Oberteil 10 ausgeübt wird, die zu einem Flexionsmoment um die Kniegelenksachse 30 führt. Eine bereits eingebeugte Stellung aufgrund des Hineinschiebens der distalen Prothesenkomponente 22 in das Unterteil 20 ist in der linken Darstellung der Figur 3 gezeigt.

In der Figur 4 wird statt einer reinen mechanischen Kopplung zwischen der distalen Prothesenkomponente 22 und dem Oberteil 10 eine hydraulische Kraftübertragungseinrichtung 40 gezeigt. Die distale Prothesenkomponente 22 ist längsverschieblich innerhalb des Unterteils 20 gelagert und kann bei einer Belastung, beispielsweise beim Auftreten während des Gehens in Richtung auf die Kniegelenksachse 30 verlagert werden. Der Verlagerungsweg ist, wie bei den übrigen Ausführungsbeispielen auch, auf eine Länge beschränkt, die für einen Prothesennutzer nicht unangenehm ist. Ein üblicher Verlagerungsweg zwischen 1 cm und 3 cm ist für einen Prothesenträger in der Regel akzeptabel. Die hydraulische Kraftübertragungseinrichtung 40 weist einen Hydraulikaktuator mit einem Gehäuse 41 auf, der an dem Unterteil 20 gelagert ist. Über eine Kolbenstange 42 ist ein beweglicher Hydraulikkolben 43 mit einem Ausleger an dem Oberteil 10 gekoppelt. Über eine Hydraulikleitung 44 ist der Hydraulikzylinder 41 mit einer Druckkammer 45 gekoppelt, die komprimierbar ist. Grundsätzlich ist es auch möglich, dass die Druckkammer 45 einen beweglich darin gelagerten Kolben aufweist. Alternativ besteht die Druckkammer 45 aus einem kompressiblen Material, beispielsweise einem elastischen Kunststoff. Die Druckkammer 45 ist dann als eine Art Puffer ausgebildet, der hydraulisch über die Hydraulikleitung 44 mit dem Hydraulikzylinder gekoppelt ist. Wird eine Kraft auf die distale Prothesenkomponente 22 ausgeübt, beispielsweise beim Auftreten, komprimiert die Druckkammer 45 und Hydraulikfluid wird durch die Hydraulikleitung 44 in das Gehäuse 41 hinein gepumpt. Dadurch wird der Hydraulikkolben 43 in proximaler Richtung in Richtung auf den Prothesenfuß 50 verlagert. Aufgrund der Anlenkung der Kolbenstange 42 an einem in Gehrichtung nach hinten weisenden Ausleger an dem Oberteil 10 erfolgt durch das Einfahren des Kolbens 43 in das Gehäuse 45 eine Flexionsbewegung und eine Einbeugung des Oberteils 10 relativ zu dem Unterteil um die Kniegelenksachse 30. Eine solche komprimierte Position sowohl der Druckkammer 45 als auch eine gebeugte Position der Protheseneinrichtung ist in der linken Darstellung gezeigt. Wird der Prothesenfuß 50 entlastet, beispielsweise Einleiten einer Schwungphase, kann durch elastische Rückstellkräfte, die durch eine entsprechend elastische Ausgestaltung der Druckkammer 45 aufgebracht werden können, das Hydraulikfluid aus dem Zylinder 41 durch die Leitung 44 in die Druckkammer 45 hineingesaugt werden, wodurch eine Bewegungsumkehr stattfindet und eine Extensionsunterstützung erfolgt. Um eine Extensionsunterstützung am Ende der Standphase oder während der Schwungphase vorzunehmen, kann im Falle einer mechanischen Kopplung gemäß der Figuren 2 und 3 auch eine Verlagerung entgegen einer Federkraft stattfindet, so dass die distale Prothesenkomponente 22 entgegen einer Feder innerhalb des Unterteils 20 verlagert wird. Bei Wegfall einer Axialbelastung in Richtung auf die Kniegelenksachse 30 wird sich eine Bewegungsumkehr einstellen, was zur Unterstützung einer Extensionsbewegung führt. Alternativ zu einer elastischen Lagerung der distalen Prothesenkomponente 22 kann auch das Oberteil 10 relativ zu dem Unterteil 20 entgegen einer Flexionsbewegung über ein elastisches Element vorgespannt sein.

Bei einer alternativen Ausgestaltung der Ausführungsform gemäß Figur 4 kann statt einer rückwärtigen Anordnung der Kolbenstange 42 an einem Ausleger auch durch entsprechende hydraulische Verschaltung ein Ausfahren der Kolbenstange 42 aus dem Zylinder 41 erfolgen, so dass die Anlenkung frontal, also in Gehrichtung vor der Kniegelenksache erfolgen kann. Durch die hydraulische Übersetzung der Kompressionsbewegung zwischen der distalen Prothesenkomponente 22 und dem Unterteil 20 kann eine Kraft- und Wegübersetzung einfach gestaltet werden, beispielsweise durch eine Variation der Kolbendurchmesser.

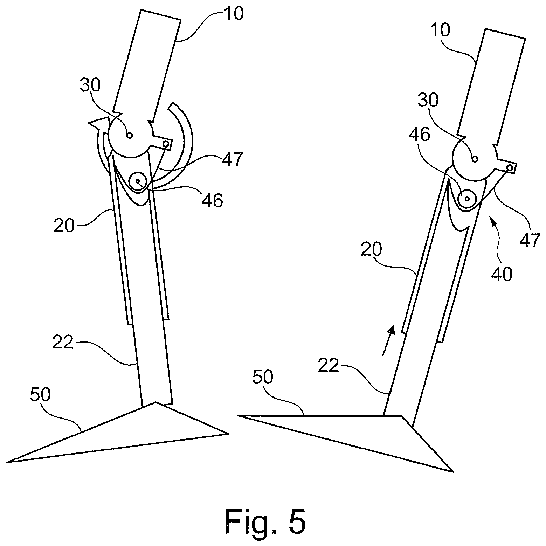

Eine weitere Ausführungsform der Erfindung ist in der Figur 5 dargestellt, bei der die Kraftübertragungseinrichtung 40 als ein Seilzug ausgebildet ist, der an der verschieblich innerhalb des Unterteils 20 gelagerten distalen Prothesenkomponente 22 angeordnet ist. In dem Ausführungsbeispiel ist die Kraftübertragungseinrichtung 40 mit einem Seil 47 ausgestattet, das an einem frontalen, proximalen Ende der distalen Prothesenkomponente 22 befestigt ist. Über eine Umlenkeinrichtung 46, beispielsweise eine Rolle oder einen Bolzen, wird das Seil 47 zu einem nach in Gehrichtung hinten ragenden Hebel an dem Oberteil 10 geführt. Wird das Unterteil 20 relativ zu der distalen Prothesenkomponente 22 verlagert, bewegt sich das proximale Ende der distalen Prothesenkomponente 22 nach oben, das Seil 47 wird aufgrund der Umlenkrolle 46 nach unten gezogen, so dass es zu einer Flexion und einem Aufbringen eines Flexionsmomentes um die Kniegelenksachse 30 kommt.

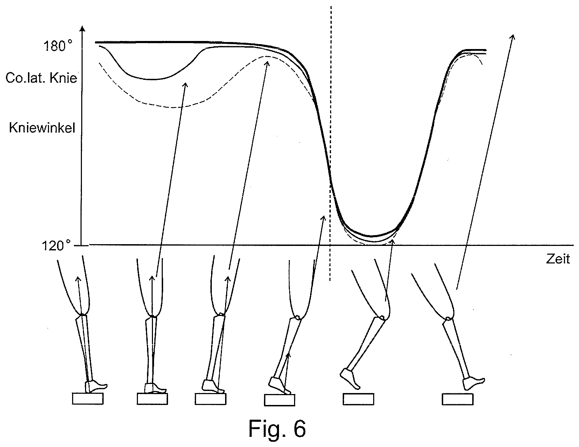

Figur 6 zeigt schematische Kniewinkelverläufe über die Zeit während des Gehens. Der obere Verlauf zeigt einen Kniewinkelverlauf mit einer Protheseneinrichtung ohne Standphasendynamik, bei der von dem Heel Strike, der ganz links dargestellt ist, bis kurz vor Ende der Standphase, die durch die gestrichelte, senkrechte Linie in dem Diagramm dargestellt ist, ein nahezu gestrecktes Prothesenkniegelenk bei einem Kniewinkel von 180° vorliegt. Die untere Kniewinkelkurve zeigt ein kontralaterales Knie, also ein unversorgtes, menschliches Kniegelenk mit einem entsprechenden Kniewinkelverlauf. Die mittlere Kurve zeigt einen beispielhaften Verlauf einer Standphasenflexionsunterstützung mit dem erfindungsgemäßen Prothesenkniegelenk, aus dem zu erkennen ist, dass eine angenähert natürliche Kniewinkelkurve während des Gehens erreicht werden kann.

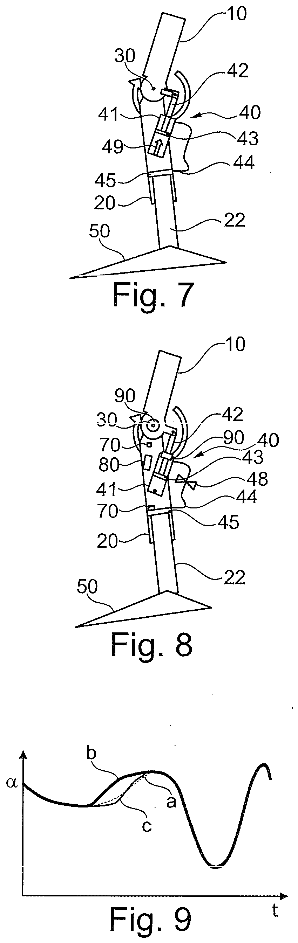

Figur 7 zeigt eine weitere Variante der Erfindung, bei der innerhalb der Kraftübertragungseinrichtung 40 zusätzlich eine Energiespeichereinrichtung 49 angeordnet ist, die innerhalb des Gehäuses 41 auf den Hydraulikkolben 43 wirkt. Die Energiespeichereinrichtung 49 speichert Flexionsenergie, also diejenige Energie, die zur Aufbringung eines Flexionsmomentes um die Kniegelenksachse 30 notwendig ist. Die Feder 25 kann eine Vorspannung aufweisen, die bevorzugt nur so groß ist, dass über die Kopplungseinrichtung 40 die gewünschte Knieflexion bei jedem Fersenauftritt eingeleitet werden kann. Die Energiespeichereinrichtung 49 stellt somit einen geringeren Widerstand gegen eine Flexion dar, als über die Kopplungseinrichtung 40 ein Flexionsmoment aufgebracht wird.

Alternativ zur Anordnung in der Kopplungseinrichtung 40 kann die Energiespeichereinrichtung 49 außerhalb der Kopplungseinrichtung 40, beispielsweise zwischen dem Oberteil 10 und dem Unterteil 20 als Biegefeder oder Knickfeder angeordnet sein. In dieser Ausgestaltung weist die Energiespeichereinrichtung 50 bevorzugt eine Federsteifigkeit auf, die sich über den Kniewinkel ändert. Insbesondere bei einem zunehmenden Beugewinkel, also bei einem verringernden Kniewinkel α, wird der Flexionswiderstand durch die Energiespeichereinrichtung 49 verringert.

In der Figur 8 ist eine weitere Variante der Erfindung dargestellt, bei der in der Hydraulikleitung 44 zwischen Extensionskammer und der Flexionskammer ein schaltbares oder verstellbares Ventil 48 angeordnet ist, sodass die Durchflussmenge von der Druckkammer 45 in die Flexionskammer beeinflusst werden kann. Das Ventil 48 wirkt als Dämpfer, der die Verschiebebewegung der distalen Prothesenkomponente 22 relativ zudem Unterteil 20 beeinflusst. Durch eine verstellbare Ausgestaltung des Ventils 48, beispielsweise motorisch oder manuell, ist eine Anpassung an dem jeweiligen Patienten oder den jeweiligen Einsatzzweck möglich. Ebenso kann der Dämpfer 60 gemäß Figur 2 einstellbar ausgebildet sein, beispielsweise durch Veränderung von Ventilstellungen in einer Leitung von der Extensionskammer in die Flexionskammer.

In der Figur 8 ist zudem ein Sensor 70 eingezeichnet, der zur Erfassung der Axialkraft, des Axialkraftverlaufs oder von Beschleunigungen ausgebildet sein kann. Statt zur Erfassung von Axialkräften oder Axialkraftverläufen kann der Sensor 70 auch zur Erfassung von Raumlagen und/oder Beschleunigungen des Unterteils 20 ausgebildet sein. Bei einer anderen Anordnung des Sensors 70 können die entsprechenden Größen für die distale Prothesenkomponente 22 erfasst werden. Ein weiterer Sensor 70 kann zur Erfassung von Winkelstellungen des Oberteils 10 relativ zum Unterteil 20 an dem Unterteil 20 oder dem Oberteil 10 angeordnet sein, in der dargestellten Ausführungsform ist der Winkelsensor 70 an dem Unterteil 20 angeordnet. Die Sensoren 70 sind mit einer Steuerungseinrichtung 80 gekoppelt, die wiederum einen Antrieb für die Drossel 48 oder das Ventil 48 ansteuert.

An dem oberen Ende des Gehäuses 41 der Kopplungseinrichtung 40 ist eine schaltbare Bremse 90 angeordnet, die mit der Steuerungseinrichtung 80 gekoppelt ist. Je nach erreichter Einbeugung in Abhängigkeit von Axialkräften oder während des Gangverlaufes ist es möglich, die Bremse 90 zu aktivieren oder zu deaktivieren. Mit einer Aktivierung der Bremse 90 ist zudem möglich, einen Extensionsanschlag sowie einen maximalen Flexionswinkel variabel oder auch permanent einzustellen. Wird beispielsweise ein Extensionsanschlag bei einem Kniewinkel von 187°, gemessen an der Rückseite der Protheseneinrichtung, erreicht, stoppt die Bremse 90 eine weitere Bewegung, entsprechend wird bei Erreichen eines maximalen Flexionswinkels die weitere Flexion gestoppt, indem die Bremse 90 aktiviert wird. Alternativ oder ergänzend kann eine Bremse 90 oder ein einstellbarer Anschlag um die Kniegelenksachse 30 herum angeordnet und ausgebildet sein.

Figur 9 zeigt drei unterschiedliche Kniewinkelverläufe über die Zeit. Die drei Kniegelenkverläufe unterscheiden sich im Wesentlich im Bereich der Standphasenflexion. Der Kniewinkelverlauf der Kurve a entspricht dem üblichen Kniewinkelverlauf bei einem gesunden, unversorgten Bein. Der Kniewinkelverlauf b steht für eine kurze Beugephase, bei der die Standphasenflexion zeitlich verkürzt ist und nach dem Heel Strike und der ersten Standphasenflexion sehr schnell ein extendiertes, gestrecktes Bein vorliegt. Ein solcher Kniewinkelverlauf kann beispielsweise durch eine starke Energiespeichereinrichtung über das Federelement 49 zur Speicherung von Flexionsenergien erreicht werden, das eine Flexionsunterstützung bereitstellt. Die dritte Kniewinkelkurve c sieht eine verzögerte Extension vor, bei der der Kniewinkel α innerhalb der Standphasenflexion für einen längeren Zeitraum verringert bleibt, verglichen mit der Kniewinkelkurve a eines gesunden Beines. Dementsprechend wird zur Erreichung des maximalen Extensionswinkels in der Standphase eine schnellere Extension als bei einem natürlichen Gelenk eingeleitet.

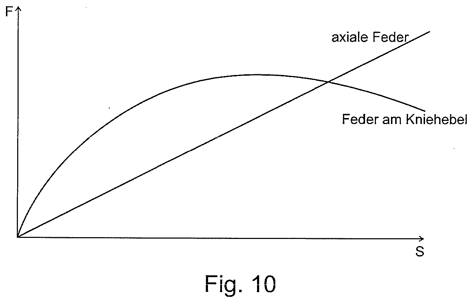

In Figur 10 ist eine Darstellung zweier Federkernlinien gezeigt, die in der Protheseneinrichtung eingesetzt werden können. Die Feder, die an einem Kniehebel ansetzt, dient zur Beeinflussung der Flexion oder Extension des Unterteils 20 relativ zu dem Oberteil 10. In der dargestellten Ausführungsform weist diese Feder über den Verformungsweg s, also bei einem abnehmenden Kniewinkel α, zunächst einen Anstieg der Federsteifigkeit bis zu einem Maximalwert auf, anschließend wird bei einer weiteren Biegung oder Verformung des Federelementes nur noch ein geringerer Widerstand bereitgestellt, sodass sich eine abfallende Federkennlinie ergibt. Dies ist beispielsweise bei einer Biegefeder zwischen dem Oberteil 10 und dem Unterteil 20 ein möglicher Knieverlauf. Eine alternative Federkernlinie ist eine lineare Federkernlinie, die mit zunehmender Verformung eine zunehmende Widerstandskraft bereitstellt, eine solche Feder kann beispielsweise als Federelement 25 gegen eine Axialverlagerung der distalen Prothesenkomponente 22 relativ zudem Unterteil 20 eingesetzt werden.

## Patentansprüche

1. Protheseneinrichtung für eine untere Extremität mit einem Prothesenkniegelenk mit einem Oberteil (10), an dem eine proximalen Prothesenkomponente (12) angeordnet ist, einem Unterteil (20), das mit dem Oberteil (10) um eine Kniegelenksachse (30) schwenkbar verbunden ist, und einer distalen Prothesenkomponente (22), an der ein Prothesenfuß (50) ausgebildet oder festlegbar ist, **dadurch gekennzeichnet, dass** die distale Prothesenkomponente (22) durch eine in Längserstreckung der distalen Prothesenkomponente (22) wirkende Axialkraft in Richtung auf die Kniegelenksachse (30) verschieblich gelagert ist und eine Kraftübertragungseinrichtung (40) dem Kniegelenk zugeordnet ist, die in der Standphase eine Verschiebung der distalen Prothesenkomponente (22) in Richtung auf die Kniegelenksachse (30) in ein Flexionsmoment um die Kniegelenksachse (30) umwandelt.

2. Protheseneinrichtung nach Anspruch 1, **dadurch gekennzeichnet, dass** die distale Prothesenkomponente (22) teleskopierbar ausgebildet oder veschieblich an dem Unterteil (20) gelagert ist.

3. Protheseneinrichtung nach Anspruch 1 oder 2, **dadurch gekennzeichnet, dass** die Kraftübertragungseinrichtung (40) als eine mechanische oder hydraulische Kraftübertragungseinrichtung (40) ausgebildet ist.

4. Protheseneinrichtung nach Anspruch 3 oder 4, **dadurch gekennzeichnet, dass** die distale Prothesenkomponente (22) über ein Federelement (25) gegen eine Verschiebung vorgespannt gelagert ist.

5. Protheseneinrichtung nach Anspruch 4, **dadurch gekennzeichnet, dass** das Federelement (25) einen progressiven Widerstand einer Verschiebung entgegensetzt.

6. Protheseneinrichtung nach einem der voranstehenden Ansprüche, **dadurch gekennzeichnet, dass** dem Kniegelenk eine Energiespeichereinrichtung (49) zur Speicherung von Flexionsenergie zugeordnet ist.

7. Protheseneinrichtung nach Anspruch 6, **dadurch gekennzeichnet, dass** die Energiespeichereinrichtung (49) als Federelement, insbesondere als Federelement mit einer sich über den Kniewinkel ändernden Federsteifigkeit, ausgebildet ist.

8. Protheseneinrichtung nach Anspruch 6 oder 7, **dadurch gekennzeichnet, dass** der Energiespeichereinrichtung (49) eine Aktivierungs- und Deaktivierungseinrichtung zugeordnet ist.

9. Protheseneinrichtung nach einem der voranstehenden Ansprüche, **dadurch gekennzeichnet, dass** dem Kniegelenk zumindest ein Dämpfer (48, 60) zur Dämpfung der Verschwenkbewegung um die Kniegelenksachse (30) und/oder der Verschiebebewegung zugordnet ist.

10. Protheseneinrichtung nach Anspruch 9, **dadurch gekennzeichnet, dass** der zumindest eine Dämpfer (48, 60) einstellbar ausgebildet ist.

11. Protheseneinrichtung nach einem der voranstehenden Ansprüche, **dadurch gekennzeichnet, dass** dem Kniegelenk eine Drossel (48) oder eine Bremse (90) zugeordnet ist, die eine Flexion um die Kniegelenksachse (30) sperrt/bremst.

12. Protheseneinrichtung nach Anspruch 11, **dadurch gekennzeichnet, dass** ein Sensor (70) mit einer Steuerungseinrichtung (80) gekoppelt ist, die einen Antrieb des Anschlages (48) oder der Bremse (90) aktiviert oder deaktiviert.

## Claims

1. A prosthetic device for a lower extremity, comprising a prosthetic knee joint having an upper part (10), on which a proximal prosthetic component (12) is arranged, a lower part (20), which is connected to the upper part (10) so to be pivotable about a knee joint axis (30), and a distal prosthetic component (22), on which a prosthetic foot (50) is formed or can be secured, **characterized in that** the distal prosthetic component (22) is mounted so as to be displaceable in the direction of the knee joint axis (30) by means of an axial force acting in the longitudinal extent of the distal prosthetic component (22), and the knee joint is assigned a force transmission device (40) which, in the stance phase, converts a displacement of the distal prosthetic component (22) in the direction of the knee joint axis (30) into a flexion moment about the knee joint axis (30).

2. The prosthetic device as claimed in claim 1, **characterized in that** the distal prosthetic component (22) is formed telescopically or mounted displaceably on the lower part (20).

3. The prosthetic device as claimed in claim 1 or 2, **characterized in that** the force transmission device (40) is in the form of a mechanical or hydraulic force transmission device (40).

4. The prosthetic device as claimed in claim 3 or 4, **characterized in that** the distal prosthetic component (22) is mounted via a spring element (25) so as to be prestressed against a displacement.

5. The prosthetic device as claimed in claim 4, **characterized in that** the spring element (25) offers progressive resistance to a displacement.

6. The prosthetic device as claimed in one of the preceding claims, **characterized in that** the knee joint is assigned an energy storage device (49) for storing flexion energy.

7. The prosthetic device as claimed in claim 6, **characterized in that** the energy storage device (49) is in the form of a spring element, in particular a spring element having a spring rigidity changing via the knee angle.

8. The prosthetic device as claimed in claim 6 or 7, **characterized in that** the energy storage device (49) is assigned an activation and deactivation device.

9. The prosthetic device as claimed in one of the preceding claims, **characterized in that** the knee joint is assigned at least one damper (48, 60) for damping the pivoting movement about the knee joint axis (30) and/or the displacement movement.

10. The prosthetic device as claimed in claim 9, **characterized in that** the at least one damper (48, 60) is adjustable.

11. The prosthetic device as claimed in one of the preceding claims, **characterized in that** the knee joint is assigned a throttle (48) or a brake (90) which blocks/brakes a flexion about the knee joint axis (30).

12. The prosthetic device as claimed in claim 11, **characterized in that** a sensor (70) is coupled to a control device (80) which activates or deactivates a drive of the stop (48) or of the brake (90).

## Revendications

1. Dispositif prothétique pour un membre inférieur, comprenant une articulation de genou prothétique comprenant une partie supérieure (10) sur laquelle est disposé un composant prothétique proximal (12), une partie inférieure (20) reliée à la partie supérieure (10) de manière à pouvoir pivoter autour d'un axe d'articulation de genou (30), et un composant prothétique distal (22) sur lequel est formé ou peut être fixé un pied prothétique (50),
**caractérisé en ce que** le composant prothétique distal (22) est monté de manière coulissante en direction de l'axe d'articulation de genou (30) par une force axiale agissant dans l'extension longitudinale du composant prothétique distal (22), et un dispositif de transmission de force (40) est associé à l'articulation de genou qui, dans la phase d'appui, transforme un coulissement du composant prothétique distal (22) en direction de l'axe d'articulation de genou (30) en un couple de flexion autour de l'axe d'articulation de genou (30).

2. Dispositif prothétique selon la revendication 1,
**caractérisé en ce que** le composant prothétique distal (22) est conçu de manière télescopique ou est monté de manière coulissante sur la partie inférieure (20).

3. Dispositif prothétique selon la revendication 1 ou 2,
**caractérisé en ce que** le dispositif de transmission de force (40) est conçu comme un dispositif de transmission de force (40) mécanique ou hydraulique.

4. Dispositif prothétique selon la revendication 3 ou 4,
**caractérisé en ce que** le composant prothétique distal (22) est monté par l'intermédiaire d'un élément élastique (25) en étant précontraint à l'encontre d'un coulissement.

5. Dispositif prothétique selon la revendication 4,
**caractérisé en ce que** l'élément élastique (25) oppose une résistance progressive au coulissement.

6. Dispositif prothétique selon l'une des revendications précédentes,
**caractérisé en ce qu'**un dispositif d'accumulation d'énergie (49) destiné à accumuler l'énergie de flexion est associé à l'articulation de genou.

7. Dispositif prothétique selon la revendication 6,
**caractérisé en ce que** le dispositif d'accumulation d'énergie (49) est réalisé sous la forme d'un élément élastique, en particulier d'un élément élastique dont la raideur varie en fonction de l'angle du genou.

8. Dispositif prothétique selon la revendication 6 ou 7,
**caractérisé en ce qu'**un dispositif d'activation et de désactivation est associé au dispositif d'accumulation d'énergie (49).

9. Dispositif prothétique selon l'une des revendications précédentes,
**caractérisé en ce qu'**au moins un amortisseur (48, 60) destiné à amortir le mouvement de pivotement autour de l'axe d'articulation de genou (30) et/ou le mouvement de coulissement est associé à l'articulation de genou.

10. Dispositif prothétique selon la revendication 9,
**caractérisé en ce que** ledit au moins un amortisseur (48, 60) est conçu de manière réglable.

11. Dispositif prothétique selon l'une des revendications précédentes,
**caractérisé en ce qu'**un étranglement (48) ou un frein (90) est associé à l'articulation de genou, qui bloque/freine une flexion autour de l'axe d'articulation de genou (30).

12. Dispositif prothétique selon la revendication 11,
**caractérisé en ce qu'**un capteur (70) est couplé à un dispositif de commande (80) qui active ou désactive un entraînement de la butée (48) ou du frein (90).
